# EUROPÄISCHE PATENTANMELDUNG

(11) **EP 4 548 958 A1**
(43) Veröffentlichungstag der Anmeldung: **07.05.2025**
(21) Anmeldenummer: 24209374.8
(22) Anmeldetag: 29.10.2024
(51) Int. Cl.: A61N 1/05, A61B 17/34, A61M 5/158, A61M 19/00, A61M 25/00, A61N 1/36

(54) **MEDIZINISCHE ANORDNUNG ZUR ELEKTRISCHEN NEUROSTIMULATION**

(30) Priorität: 03.11.2023 DE 102023130417
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Die Erfindung betrifft eine medizinische Anordnung zur elektrischen Neurostimulation, aufweisend eine Einführhilfe mit einem längserstreckten Einführschaft, der ein proximales Schaftende, ein distales Schaftende und ein zwischen dem proximalen Schaftende und dem distalen Schaftende längserstrecktes Lumen aufweist, einen Stimulator mit einem längserstreckten Stimulatorschaft, der ein distales Stimulatorende mit wenigstens einer Stimulationselektrode aufweist, die zur Abgabe von Stromimpulsen eingerichtet ist, und der einen elektrischen Leiter aufweist, dessen distales Ende die wenigstens eine Stimulationselektrode bildet oder mit der wenigstens einen Stimulationselektrode verbunden ist, wobei die Anordnung zwischen einer ersten Konfiguration und einer zweiten Konfiguration überführbar ist, wobei in der ersten Konfiguration der Stimulatorschaft über das proximale Schaftende des Einführschafts in das Lumen eingeführt ist und das distale Stimulatorende über das distale Schaftende hinausragt, und wobei in der zweiten Konfiguration der Stimulatorschaft aus dem Lumen des Einführschafts proximal herausgezogen ist und die Einführhilfe und der Stimulator voneinander getrennt sind.

## Beschreibung

Die Erfindung betrifft eine medizinische Anordnung zur elektrischen Neurostimulation.

Die elektrische Neurostimulation (kurz: Neurostimulation oder Neuromodulation) ist eine etablierte Therapieform zur Behandlung akuter oder chronischer Schmerzen und sieht die Abgabe von Stromimpulsen in unmittelbarer Nähe eines Nervs vor, um dessen Leitungsverhalten für Schmerzimpulse zu beeinflussen.

Aufgabe der Erfindung ist es, eine medizinische Anordnung zur elektrischen Neurostimulation bereitzustellen, die einen einfachen Aufbau aufweist und vielseitig verwendbar ist.

Diese Aufgabe wird durch das Bereitstellen einer medizinischen Anordnung mit den Merkmalen des Anspruchs 1 gelöst. Vorteilhafte Weiterbildungen sind in den Unteransprüchen angegeben.

Die erfindungsgemäße medizinische Anordnung ist zur elektrischen Neurostimulation eingerichtet und weist eine Einführhilfe und einen Stimulator auf. Die Einführhilfe weist einen längserstreckten Einführschaft mit einem proximalen Schaftende, einem distalen Schaftende und einem zwischen dem proximalen Schaftende und dem distalen Schaftende längserstreckten Lumen auf. Der Stimulator weist einen längserstreckten Stimulatorschaft mit einem distalen Stimulatorende und einem elektrischen Leiter auf. Das distale Stimulatorende weist wenigstens eine Stimulationselektrode auf, die zur Abgabe von Stromimpulsen eingerichtet ist. Ein distales Ende des elektrischen Leiters ist mit der wenigstens einen Stimulationselektrode verbunden oder bildet diese. Die erfindungsgemäße Anordnung ist zwischen einer ersten Konfiguration und einer zweiten Konfiguration überführbar. In der ersten Konfiguration ist der Stimulatorschaft über das proximale Schaftende des Einführschafts in das Lumen eingeführt und das distale Stimulatorende ragt über das distale Schaftende hinaus. In der zweiten Konfiguration ist der Stimulatorschaft aus dem Lumen des Einführschafts proximal herausgezogen und die Einführhilfe und der Stimulator sind voneinander getrennt. Die erste Konfiguration dient der Neurostimulation mittels der besagten Abgabe der Stromimpulse über die wenigstens eine Stimulationselektrode des Stimulators. In der zweiten Konfiguration kann ein Lokalanästhetikum über das Lumen der Einführhilfe abgegeben werden. Die Einführhilfe weist folglich eine vorteilhafte Mehrfachfunktion auf und dient zum einen einem vereinfachten Einführen des Stimulators und zum anderen als eine Art Kapillar oder auch Katheter zur Abgabe des Lokalanästhetikums. Durch die funktionale Trennung zwischen Neurostimulation einerseits und Abgabe des Lokalanästhetikums andererseits kann der Stimulator möglichst einfach aufgebaut werden. Dabei weist der Stimulator vorzugsweise kein Lumen zur Abgabe eines Lokalanästhetikums auf. Das Lumen der Einführhilfe kann alternativ oder zusätzlich zu der besagten Abgabe des Lokalanästhetikums und/oder zur Aufnahme eines Katheters eingerichtet sein, der zur Abgabe eines/des Lokalanästhetikums eingerichtet ist.

In Ausgestaltung der Erfindung besteht der Stimulatorschaft aus dem elektrischen Leiter, wobei der Stimulatorschaft keine elektrische Isolation aufweist, und wobei in der ersten Konfiguration der Einführschaft eine elektrisch isolierende Ummantelung für den elektrischen Leiter bildet. Diese Ausgestaltung erlaubt eine weiter vereinfachte Gestaltung des Stimulators. Die vereinfachte Gestaltung wird dadurch erreicht, dass der Stimulatorschaft aus dem elektrischen Leiter besteht. Das distale Ende des elektrischen Leiters bildet die wenigstens eine Stimulationselektrode. Der Stimulatorschaft/elektrische Leiter weist selbst keine elektrische Isolation auf. Die Isolationsfunktion wird von dem Einführschaft übernommen, der in der ersten Konfiguration der medizinischen Anordnung gleichsam eine elektrisch isolierende Ummantelung für den elektrischen Leiter bildet.

In weiterer Ausgestaltung der Erfindung weist der Stimulatorschaft eine elektrisch isolierende Ummantelung auf, die den elektrischen Leiter abschnittsweise ummantelt, wobei das distale Stimulatorende nicht von dem elektrischen Leiter ummantelt ist, und wobei das in der ersten Konfiguration über das distale Schaftende des Einführschafts hinausragende Stimulatorende einen ersten Endabschnitt, der nicht von der Ummantelung ummantelt ist und die wenigstens eine Stimulationselektrode aufweist, und einen zweiten Endabschnitt aufweist, der von der Ummantelung ummantelt ist. Durch die Unterteilung des Stimulatorendes in den ersten Endabschnitt und den zweiten Endabschnitt kann eine präzise positionierte Abgabe der Stromimpulse erfolgen. Die Abgabe erfolgt über die wenigstens eine an dem ersten Endabschnitt angeordnete oder ausgebildete Stimulationselektrode. Zu diesem Zweck ist der erste Endabschnitt nicht von der elektrisch isolierenden Ummantelung ummantelt. Der zweite Endabschnitt ist stattdessen elektrisch isoliert. Beide Endabschnitte ragen in der ersten Konfiguration distal über das distale Schaftende des Einführschafts hinaus.

In weiterer Ausgestaltung der Erfindung weist das distale Stimulatorende mehrere Stimulationselektroden auf, die zur separaten Abgabe von Stromimpulsen eingerichtet sind. Die mehreren Stimulationselektroden zur separaten Abgabe von Stromimpulsen erlauben eine Optimierung der Neurostimulation. Bei einer Ausgestaltung sind die mehreren Stimulationselektroden proximodistal und/oder entlang einer Längsachse des Stimulatorschafts voneinander beabstandet an dem distalen Stimulatorende angeordnet oder ausgebildet. Bei einer weiteren Ausgestaltung sind die mehreren Stimulationselektroden alternativ oder zusätzlich in Umfangsrichtung des distalen Stimulatorendes voneinander beabstandet angeordnet oder ausgebildet. Bei einer Ausgestaltung bildet wenigstens eine der mehreren Stimulationselektroden eine Gegenelektrode für die verbleibenden Stimulationselektroden.

In weiterer Ausgestaltung der Erfindung ist wenigstens eine Gegenelektrode vorhanden, die als Hautelektrode zum Aufbringen auf die Haut eines Patienten gestaltet oder an dem Stimulatorende angeordnet ist. Die wenigstens eine Gegenelektrode dient als Gegenpol für die wenigstens eine Stimulationselektrode. Zur Abgabe der Stromimpulse wird zwischen der wenigstens einen Stimulationselektrode und der Gegenelektrode eine Spannungsdifferenz erzeugt. Bei einer Ausgestaltung ist die wenigstens eine Gegenelektrode eine Hautelektrode zum Aufbringen auf die Haut. Solche Hautelektroden sind dem Fachmann bekannt und in unterschiedlichen Ausführungen am Markt erhältlich. Durch die Verwendung einer solchen Hautelektrode als Gegenelektrode kann der Aufbau der medizinischen Anordnung weiter vereinfacht werden. Bei einer weiteren Ausgestaltung ist die wenigstens eine Gegenelektrode an dem Stimulatorende angeordnet. Sofern das Stimulatorende mehrere Elektroden aufweist, kann eine der mehreren Elektroden als Gegenelektrode fungieren.

In weiterer Ausgestaltung der Erfindung weist der Stimulatorschaft ein zwischen dem distalen Stimulatorende und einem proximalen Stimulatorende längserstrecktes Lumen auf, das zur Abgabe eines Lokalanästhetikums eingerichtet ist. Hierdurch kann der Stimulator zum einen zur Neuromodulation und zum anderen zur Abgabe des Lokalanästhetikums verwendet werden. Dies erlaubt zwar eine vielseitigere Verwendung der medizinischen Anordnung, wobei aber der Aufbau des Stimulators im Vergleich zu Ausgestaltungen ohne Lumen weniger einfach und damit weniger vorteilhaft ist.

In weiterer Ausgestaltung der Erfindung ist eine Steuereinrichtung vorhanden und mit dem elektrischen Leiter des Stimulatorschafts verbunden oder verbindbar, wobei die Steuereinrichtung zum Steuern der Abgabe der Stromimpulse über die wenigstens eine Stimulationselektrode eingerichtet ist. Bei einer Ausgestaltung der Erfindung ist die Steuereinrichtung integraler Bestandteil des Stimulators. Bei einer weiteren Ausgestaltung ist die Steuereinrichtung eine separate Einheit und elektrisch mit dem Stimulator verbindbar oder verbunden.

In weiterer Ausgestaltung der Erfindung ist die Steuereinrichtung mit den mehreren Stimulationselektroden verbunden oder verbindbar, wobei die Steuereinrichtung zum Steuern der Abgabe der separaten Stromimpulse eingerichtet ist. Bei dieser Ausgestaltung der Erfindung ist die Steuereinrichtung zum separaten Ansteuern der mehreren Stimulationselektroden eingerichtet. Dies erlaubt eine Optimierung der schmerzreduzierenden Wirkung der Neuromodulation. Die Steuereinrichtung ist über den elektrischen Leiter des Stimulatorschafts mit den mehreren Stimulationselektroden verbunden oder verbindbar. Es versteht sich, dass der Stimulatorschaft auch mehrere elektrische Leiter aufweisen kann, wobei jeweils einer der mehreren elektrischen Leiter einer der mehreren Stimulationselektroden zugeordnet ist.

In weiterer Ausgestaltung der Erfindung ist ein Katheter vorhanden und zur Abgabe eines Lokalanästhetikums eingerichtet, wobei die medizinische Anordnung in eine dritte Konfiguration überführbar ist, in welcher der Katheter anstelle des Stimulatorschafts in das Lumen der Einführhilfe eingeführt ist. Der Katheter kann auch als Schmerzkatheter bezeichnet werden und weist einen dem Fachmann bekannten Aufbau auf. Durch die Ergänzung der medizinischen Anordnung um den besagten Katheter kann eine noch vielseitigere Verwendbarkeit erreicht werden. Insbesondere kann auf eine spezifische Gestaltung der Einführhilfe, die eine Abgabe des Lokalanästhetikums über das Lumen des Einführschafts erlaubt, verzichtet werden.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch vereinfachter Darstellung eine Ausführungsform einer medizinischen Anordnung zur elektrischen Neurostimulation mit einer im Längsschnitt dargestellten Einführhilfe und einem Stimulator, wobei die Einführhilfe und der Stimulator proximal abgeschnitten dargestellt sind,
- Fig. 2: die medizinische Anordnung nach Fig. 1, wobei der Stimulator distal in die Einführhilfe eingeschoben ist,
- Fig. 3: in schematischer Blockdarstellung eine Steuereinrichtung der medizinischen Anordnung nach den Fig. 1 und 2,
- Fig. 4: eine weitere Ausführungsform einer erfindungsgemäßen medizinischen Anordnung in einer Ansicht entsprechend Fig. 2,
- Fig. 5: eine weitere Ausführungsform einer erfindungsgemäßen medizinischen Anordnung in einer Ansicht entsprechend Fig. 2,
- Fig. 6: eine weitere Ausführungsform einer erfindungsgemäßen medizinischen Anordnung in einer Ansicht entsprechend Fig. 2,
- Fig. 7: eine weitere Ausführungsform einer erfindungsgemäßen medizinischen Anordnung in einer Ansicht entsprechend Fig. 2,
- Fig. 8: in schematischer Blockdarstellung eine Gegenelektrode der medizinischen Anordnungen nach den Fig. 1 bis 7,
- Fig. 9: eine weitere Ausführungsform eines erfindungsgemäßen Stimulators mit einem Lumen, das teilweise freigeschnitten dargestellt ist, und
- Fig. 10: die Einführhilfe der Anordnung nach den Fig. 1 und 2 zusammen mit einem Katheter, der anstelle des Stimulators in die Einführhilfe eingeschoben ist.

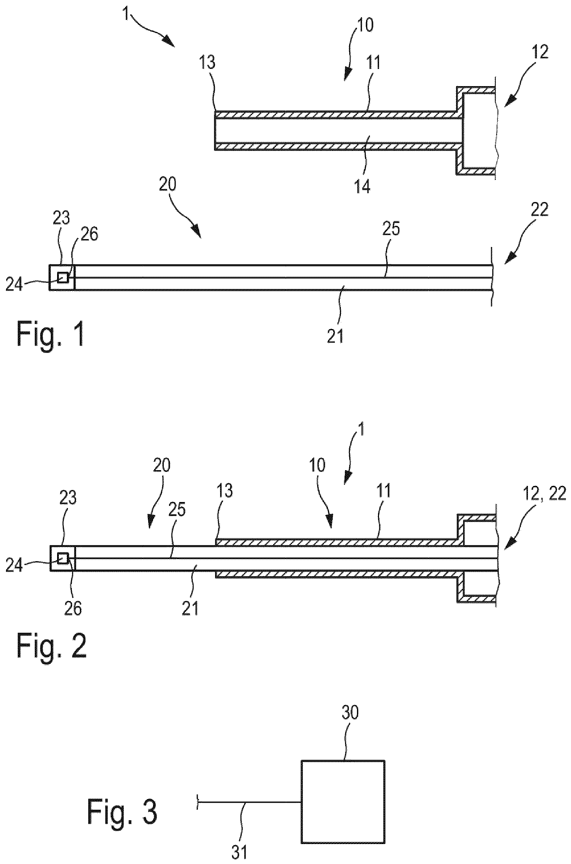

Gemäß den Fig. 1 und 2 ist eine medizinische Anordnung 1 zur elektrischen Neurostimulation bei einer Schmerztherapie vorgesehen und weist eine Einführhilfe 10 und einen Stimulator 20 auf.

Die Einführhilfe 10 weist einen längserstreckten Einführschaft 11 mit einem proximalen Schaftende 12 und einem distalen Schaftende 13 auf. Zur vereinfachten Darstellung ist der Einführschaft 11 im Bereich des proximalen Schaftendes 12 abgeschnitten dargestellt. Zwischen dem proximalen Schaftende 12 und dem distalen Schaftende 13 weist der Einführschaft 11 ein längserstrecktes Lumen 14 auf. Die Einführhilfe 10 kann auch als Kapillar bezeichnet werden.

Der Stimulator 20 weist einen längserstreckten Stimulatorschaft 21 mit einem proximalen Stimulatorende 22 und einem distalen Stimulatorende 23 auf. Der Stimulatorschaft 21 ist zwecks vereinfachter Darstellung im Bereich des proximalen Stimulatorendes 22 abgeschnitten dargestellt. Das distale Stimulatorende 23 weist wenigstens eine Stimulationselektrode 24 auf, die zur Abgabe von Stromimpulsen eingerichtet ist. Weiter weist der Stimulatorschaft 21 einen elektrischen Leiter 25 auf. Dessen distales Ende 26 ist vorliegend mit der wenigstens einen Stimulationselektrode 24 verbunden. Alternativ kann das distale Ende die Stimulationselektrode bilden.

In Fig. 2 ist die medizinischen Anordnung 1 in einer ersten Konfiguration gezeigt, in welcher der Stimulatorschaft 21 über das proximale Schaftende 12 des Einführschafts 11 in das Lumen 14 eingeführt ist und das distale Stimulatorende 23 über das distale Schaftende 13 hinausragt.

In Fig. 1 ist die medizinische Anordnung 1 in einer zweiten Konfiguration gezeigt, in welcher der Stimulatorschaft 21 aus dem Lumen 14 des Einführschafts 11 proximal herausgezogen ist und die Einführhilfe 10 und der Stimulator voneinander getrennt sind.

Die erste Konfiguration (Fig. 2) dient der Neuromodulation mittels der besagten Abgabe von Stromimpulsen über die wenigstens eine Stimulationselektrode 24. Zu diesem Zweck wird das distale Stimulatorende 23 mitsamt der wenigstens einen Stimulationselektrode 24 in unmittelbarer Nähe eines zu betäubenden Nervs platziert. Das Leitungsverhalten des Nervs für Schmerzimpulse wird durch die von der Stimulationselektrode 24 abgegebenen Stromimpulse beeinflusst. Dieser Therapieansatz zur Behandlung akuter oder chronischer Schmerzen ist dem Fachmann bekannt.

In der zweiten Konfiguration (Fig. 1) kann die Einführhilfe 10 zur Verabreichung eines Lokalanästhetikums verwendet werden. Zu diesem Zweck kann das Lokalanästhetikum über das proximale Schaftende 12 in das Lumen 14 eingeleitet und über das distale Schaftende 13 lokal begrenzt an den zu betäubenden Nerv abgegeben werden. Das proximale Schaftende 12 kann mit einem Fluidkonnektor, einem Zuspritzport oder dergleichen versehen sein.

Zur Ansteuerung der wenigstens einen Stimulationselektrode 24 weist die medizinische Anordnung 1 zudem eine Steuereinrichtung 30 auf. Die Steuereinrichtung 30 ist als Funktionsblock schematisch vereinfacht in Fig. 3 gezeigt und über eine Steuerleitung 31 mit dem elektrischen Leiter 25 verbunden oder verbindbar. Die Steuereinrichtung 30 ist zum Steuern der Abgabe der Stromimpulse über die wenigstens eine Stimulationselektrode 24 eingerichtet.

Bei der in den Fig. 1 bis 3 gezeigten Ausführungsform ist die Steuereinrichtung 30 örtlich getrennt von dem Stimulator 20 dargestellt und bildet eine separate Einheit. Bei einer in den Figuren nicht gezeigten Ausführungsform ist die Steuereinrichtung in den Stimulator integriert.

In den weiteren Figuren sind weitere Ausführungsformen medizinischer Anordnungen 1a, 1b, 1c, 1d gezeigt. Die weiteren Anordnungen 1a bis 1d sind hinsichtlich ihrer Struktur und Funktion im Wesentlichen identisch mit der Anordnung 1. Zur Vermeidung von Wiederholungen werden daher nachfolgend in erster Linie wesentliche Unterschiede der Anordnungen 1a bis 1d gegenüber der Anordnung 1 erläutert. Im Übrigen und sofern nicht abweichend beschrieben, gilt das zu der Anordnung 1 Offenbarte, mutatis mutandis, auch für die weiteren Anordnungen 1a bis 1d. Funktionsgleiche Bauteile und/oder Abschnitte sind mit identischen Bezugszeichenziffern unter Hinzufügung von Kleinbuchstaben gekennzeichnet.

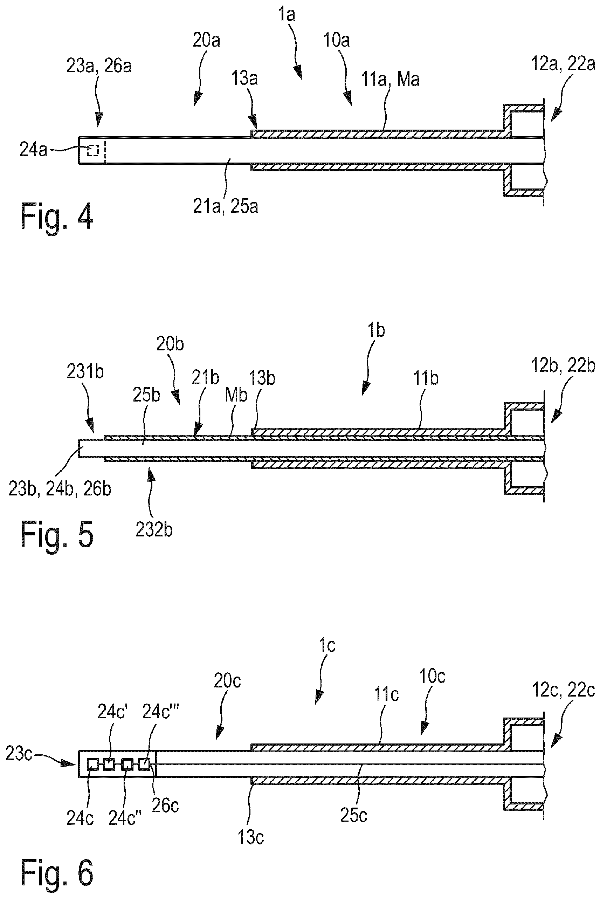

Bei der medizinischen Anordnung 1a nach Fig. 4 besteht der Stimulatorschaft 21a aus dem elektrischen Leiter 25a. Der Stimulatorschaft 21a/der elektrische Leiter 25a selbst weist keine elektrische Isolation auf. Stattdessen bildet der Einführschaft 11a in der ersten Konfiguration, wie sie in Fig. 4 gezeigt ist, eine elektrisch isolierende Ummantelung Ma für den elektrischen Leiter 25a. Das distale Ende 26a des elektrischen Leiters 25a bildet das distale Stimulatorende 23a. Zudem bildet der elektrische Leiter 25a im Bereich seines distalen Endes 26a gleichzeitig wenigstens eine Stimulationselektrode 24a aus. Das distale Stimulatorende 23a und die Stimulationselektrode 24a sind in Fig. 4 mit strichlierten Linien angedeutet, wobei die gezeigte Formgebung und Abmessung als rein exemplarisch zu verstehen ist. Prinzipiell bildet der gesamte über das distale Schaftende 13a hinausragende Anteil des elektrischen Leiters 25a eine/die Stimulationselektrode.

Bei der medizinischen Anordnung 1b nach Fig. 5 weist der Stimulatorschaft 21b eine elektrisch isolierende Ummantelung Mb auf, die den elektrischen Leiter 25b wenigstens abschnittsweise ummantelt. Im Bereich des distalen Stimulatorendes 23b liegt der elektrische Leiter 25b frei. Dort ist keine Ummantelung vorhanden. In der ersten Konfiguration der medizinischen Anordnung 1b, wie sie in Fig. 5 gezeigt ist, weist das über das distale Schaftende 13b hinausragende Stimulatorende 23b einen ersten (nicht isolierten) Endabschnitt 231b und einen zweiten (elektrisch isolierten) Endabschnitt 232b auf. Der erste Endabschnitt 231b bildet die wenigstens eine Stimulationselektrode 24b.

Bei der medizinischen Anordnung 1c nach Fig. 6 weist das distale Stimulatorende 23c mehrere Stimulationselektroden 24c, 24c`, 24c", 24c‴ auf. Diese sind zur separaten Abgabe von Stromimpulsen eingerichtet. Durch die separate Abgabe der Stromimpulse über die mehreren Stimulationselektroden 24c, 24c`, 24c", 24‴ kann eine örtlich gezielte Abgabe der Stromimpulse und damit eine Optimierung der Neurostimulation im Hinblick auf die erzielbare Schmerzreduktion erreicht werden. Bei der in Fig. 6 gezeigten Ausführungsform sind die mehreren Stimulationselektroden 24c, 24c`, 24c", 24‴ proximodistal voneinander beabstandet an dem distalen Stimulatorende 23c angeordnet. Alternativ oder zusätzlich können die mehreren Stimulationselektroden 24c, 24c`, 24c", 24‴ in Umfangsrichtung des distalen Stimulatorendes 23c voneinander beabstandet sein. Die elektrische Kontaktierung der mehreren Stimulationselektroden 24c, 24c`, 24c", 24‴ erfolgt mittels des elektrischen Leiters 25c. Es versteht sich, dass der Stimulatorschaft 21c mehrere elektrische Leiter aufweisen kann, wobei jeweils einer der elektrischen Leiter zur Kontaktierung und/oder Ausbildung einer der Stimulationselektroden dient.

Weiter versteht sich, dass die Steuereinrichtung 30 nach Fig. 3 auch bei den medizinischen Anordnungen 1a, 1b, 1c, 1d vorhanden sein kann. Sofern mehrere Stimulationselektroden vorhanden sind, ist die Steuereinrichtung 30 vorzugsweise zum Steuern der Abgabe der separaten Stromimpulse über die mehreren Stimulationselektroden eingerichtet.

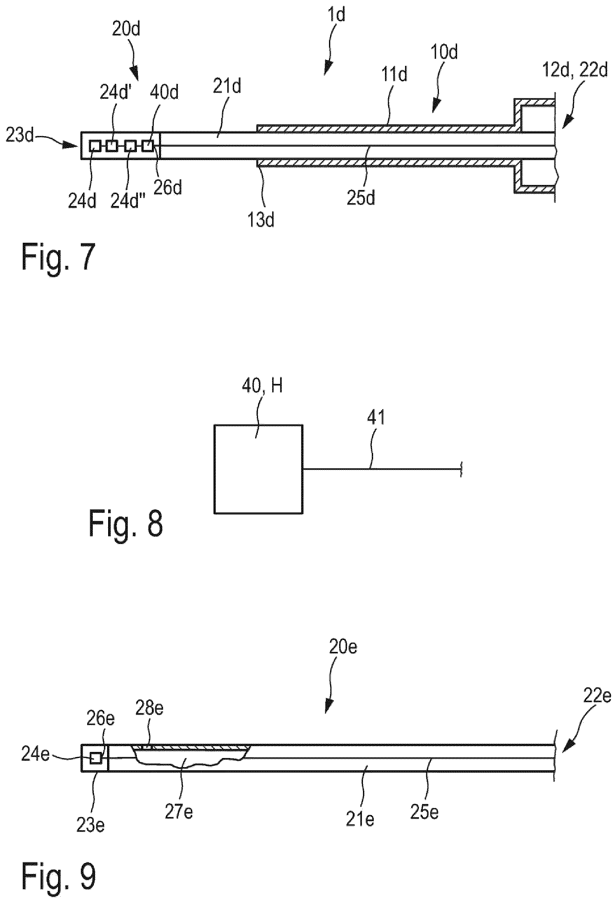

Bei der medizinischen Anordnung 1d nach Fig. 7 ist eine Gegenelektrode 40d vorhanden und an dem distalen Stimulatorende 23d angeordnet. Die Gegenelektrode 40d kann über einen gesonderten elektrischen Leiter oder über einen gesonderten Leitungsstrang des elektrischen Leiters 25d elektrisch kontaktiert sein. Die Gegenelektrode 40d und die mehreren Stimulationselektroden 24d, 24d`, 24d" weisen unterschiedliche elektrische Polaritäten auf.

Fig. 8 zeigt eine Gegenelektrode 40 in Form einer Hautelektrode H. Die Hautelektrode H ist zum Aufbringen auf die Haut eines Patienten eingerichtet und über eine Signalleitung 41 mit der Steuereinrichtung 30 verbunden oder verbindbar. Die Gegenelektrode 40 kann Bestandteil der medizinischen Anordnungen 1, 1a, 1b, 1c sein.

Fig. 9 zeigt einen Stimulator 20e, der im Unterschied zu den Stimulatoren 20, 20a, 20b, 20c, 20d ein Lumen 27e mit wenigstens einer Auslassöffnung 28e aufweist. Das Lumen 27e ist zwischen dem proximalen Stimulatorende 22e und dem distalen Stimulatorende 23e längserstreckt und mündet einends in die Auslassöffnung 28e. Das Lumen 27e ist zur Abgabe eines Lokalanästhetikums durch die Auslassöffnung 28e eingerichtet.

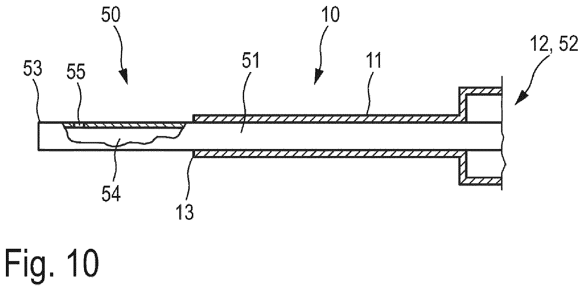

Fig. 10 zeigt die Anordnung nach den Fig. 1 und 2 in einer dritten Konfiguration. In der dritten Konfiguration ist anstelle des Stimulators 20 ein Katheter 50 in das Lumen 14 des Einführschafts 11 eingeschoben. Der Katheter 50 weist einen Katheterschaft 51 mit einem proximalen Katheterende 52 und einem distalen Katheterende 53 auf. Weiter weist der Katheterschaft 51 ein zwischen dem proximalen Katheterende 52 und dem distalen Katheterende 53 längserstrecktes Lumen 54 mit wenigstens einer Auslassöffnung 55 auf. Der Katheter 50 kann auch als Schmerzkatheter bezeichnet werden und dient der Abgabe eines Lokalanästhetikums über das Lumen 54 durch die Auslassöffnung 55.

Es versteht sich, dass einzelne Merkmale der vorhergehend erläuterten Ausführungsformen unter Ausbildung weiterer Ausführungsformen miteinander kombinierbar sind.

## Patentansprüche

1. Medizinische Anordnung (1, 1a bis 1d) zur elektrischen Neurostimulation, aufweisend
eine Einführhilfe (10, 10a bis 10d) mit einem längserstreckten Einführschaft (11, 11a bis 11d), der ein proximales Schaftende (12, 12a bis 12d), ein distales Schaftende (13, 13a bis 13d) und ein zwischen dem proximalen Schaftende (12, 12a bis 12d) und dem distalen Schaftende (13, 13a bis 13d) längserstrecktes Lumen (14) aufweist,
einen Stimulator (20, 20a bis 20e) mit einem längserstreckten Stimulatorschaft (21, 21a bis 21e), der ein distales Stimulatorende (23, 23a, 23e) mit wenigstens einer Stimulationselektrode (24, 24a bis 24e) aufweist, die zur Abgabe von Stromimpulsen eingerichtet ist, und der einen elektrischen Leiter (25, 25a bis 25e) aufweist, dessen distales Ende (26, 26a bis 26e) die wenigstens eine Stimulationselektrode (24, 24a bis 24e) bildet oder mit der wenigstens einen Stimulationselektrode (24, 24a bis 24e) verbunden ist,
wobei die medizinische Anordnung (1, 1a bis 1d) zwischen einer ersten Konfiguration und einer zweiten Konfiguration überführbar ist,
wobei in der ersten Konfiguration der Stimulatorschaft (21, 21a bis 21e) über das proximale Schaftende (12, 12a bis 12d) des Einführschafts (11, 11a bis 11d) in das Lumen (14) eingeführt ist und das distale Stimulatorende (23, 23a, 23e) über das distale Schaftende (13, 13a bis 13d) hinausragt, und
wobei in der zweiten Konfiguration der Stimulatorschaft (21, 21a bis 21e) aus dem Lumen (14) des Einführschafts (11, 11a bis 11d) proximal herausgezogen ist und die Einführhilfe (10, 10a bis 10d) und der Stimulator (20, 20a bis 20e) voneinander getrennt sind.

2. Medizinische Anordnung (1a) nach Anspruch 1, **dadurch gekennzeichnet, dass** der Stimulatorschaft (21a) aus dem elektrischen Leiter (25a) besteht, wobei der Stimulatorschaft (21a) keine elektrische Isolation aufweist, und wobei in der ersten Konfiguration der Einführschaft (11a) eine elektrisch isolierende Ummantelung (Ma) für den elektrischen Leiter (25a) bildet.

3. Medizinische Anordnung (1b) nach Anspruch 1, **dadurch gekennzeichnet, dass** der Stimulatorschaft (21b) eine elektrisch isolierende Ummantelung (Mb) aufweist, welche den elektrischen Leiter (25b) abschnittsweise ummantelt, wobei das in der ersten Konfiguration über das distale Schaftende (13b) hinausragende distale Stimulatorende (23b) einen ersten Endabschnitt (231b), der nicht von der Ummantelung (Mb) ummantelt ist und die wenigstens eine Stimulationselektrode (24b) aufweist, und einen zweiten Endabschnitt (232b) aufweist, der von der Ummantelung (Mb) ummantelt ist.

4. Medizinische Anordnung (1c, 1d) nach einem der vorhergehenden Ansprüche, **dadurch gekennzeichnet, dass** das distale Stimulatorende (23c, 23d) mehrere Stimulationselektroden (24c, 24c`, 24c", 24c‴; 24d, 24d`, 24d") aufweist, die zur separaten Abgabe von Stromimpulsen eingerichtet sind.

5. Medizinische Anordnung (1, 1a bis 1d) nach einem der vorhergehenden Ansprüche, **dadurch gekennzeichnet, dass** wenigstens eine Gegenelektrode (40, 40d) vorhanden ist, die als Hautelektrode (H) zum Aufbringen auf die Haut eines Patienten gestaltet oder an dem distalen Stimulatorende angeordnet ist.

6. Medizinische Anordnung (1, 1a bis 1d) nach einem der vorhergehenden Ansprüche, **dadurch gekennzeichnet, dass** der Stimulatorschaft (21e) ein zwischen dem distalen Stimulatorende (23e) und einem proximalen Stimulatorende (22e) längserstrecktes Lumen (27e) aufweist, das zur Abgabe eines Lokalanästhetikums eingerichtet ist.

7. Medizinische Anordnung (1, 1a bis 1d) nach einem der vorhergehenden Ansprüche, **dadurch gekennzeichnet, dass** eine Steuereinrichtung (30) vorhanden und mit dem elektrischen Leiter (25, 25a bis 25e) des Stimulators (20, 20a bis 20e) verbunden oder verbindbar ist, wobei die Steuereinrichtung (30) zum Steuern der Abgabe der Stromimpulse über die wenigstens eine Stimulationselektrode (24, 24a bis 24e) eingerichtet ist.

8. Medizinische Anordnung (1c, 1d) nach Anspruch 7 und Anspruch 4, **dadurch gekennzeichnet, dass** die Steuereinrichtung (30) mit den mehreren Stimulationselektroden (24c, 24c`, 24c", 24c‴; 24d, 24d`, 24d") verbunden oder verbindbar ist, wobei die Steuereinrichtung (30) zum Steuern der Abgabe der separaten Stromimpulse eingerichtet ist.

9. Medizinische Anordnung (1, 1a bis 1d) nach einem der vorhergehenden Ansprüche, **dadurch gekennzeichnet, dass** ein Katheter (50) vorhanden und zur Abgabe eines Lokalanästhetikums eingerichtet ist, wobei die medizinische Anordnung (1, 1a bis 1d) in eine dritte Konfiguration überführbar ist, in welcher der Katheter (50) anstelle des Stimulatorschafts (21, 21a bis 21e) in das Lumen (14) der Einführhilfe (10, 10a bis 10d) eingeführt ist.
